# EUROPÄISCHE PATENTSCHRIFT

(11) **EP 1 388 528 B1**
(45) Veröffentlichungstag und Bekanntmachung des Hinweises auf die Patenterteilung: **08.04.2015**
(21) Anmeldenummer: 03013917.4
(22) Anmeldetag: 20.06.2003
(51) Int. Cl.: C07C 2/28, C07C 7/177, C07C 11/08

(54) **Verfahren zur Oligomerisierung von Isobuten in n-Buten-haltigen Kohlenwasserstoffströmen**
Process for the oligomerisation of isobutene contained in hydrocarbon streams containing n-butene
Procédé pour l'oligomérisation d'isobutène contenu dans des courants d'hydrocarbures contenant du n-butène

(30) Priorität: 06.08.2002 DE 10235934; 13.02.2003 DE 10306214
(43) Veröffentlichungstag der Anmeldung: 11.02.2004
(73) Patentinhaber: Evonik Degussa GmbH, 45128 Essen (DE)
(72) Erfinder: Beckmann, Andreas, Dr., 45657 Recklinghausen (DE); Nierlich, Franz, Dr., 45768 Marl (DE); Peters, Udo, Dr., 45770 Marl (DE); Büschken, Wilfried, Dr., 45721 Haltern am See (DE); Kerker, Lothar, Dr., 48249 Dülmen (DE); Maschmeyer, Dietrich, Dr., 45657 Recklinghausen (DE); Röttger, Dirk, Dr., 45657 Recklinghausen (DE)
(74) Vertreter: Hirsch, Hans-Ludwig

(56) Entgegenhaltungen:
- WO-A-01/51435
- WO-A-02/064531

## Beschreibung

Die Erfindung betrifft ein Verfahren zur Oligomerisation von Isobuten in Isobuten-haltigen Kohlenwasserstoffgemischen an einem festen sauren Ionenaustauscherharz, wobei das saure Ionenaustauscherharz Sulfonsäuregruppen enthält, deren Protonen teilweise gegen Metallionen ausgetauscht wurden.

Ein Gemisch aus 2,4,4-Trimethylpent-1-en und 2,4,4-Trimethylpent-2-en, technisch meist als Diisobuten bezeichnet, wird großtechnisch durch Dimerisierung von Isobuten gewonnen. Es ist, insbesondere nach Hydrierung zum entsprechenden Paraffin (Isooktan, 2,2,4-Trimethylpentan ) wegen seiner hohen Octanzahl (ein Maß für die Klopffestigkeit des Vergaserkraftstoffs) eine begehrte Kraftstoffkomponente. Isooktan dient auch als Referenz zur Bestimmung der Oktanzahl. Für den Einsatz als Kraftstoffadditiv können auch Mischungen des Diisobutens bzw. Isooktans genutzt werden, die andere C₈-Isomere oder Kohlenwasserstoffe mit anderen C-Zahlen enthalten.

Für die Verwendung in Synthesen sind normalerweise höhere Reinheiten des Diisobutens erwünscht. Durch Hydroformylierung des Diisobutens wird 3,5,5-Trimethylhexanal erhalten, das zur entsprechenden Carbonsäure oxidiert werden kann. Die Carbonsäure findet Einsatz zur Herstellung von Peroxiden, Schmiermittel und Sikkativen. Weiterhin wird Diisobuten zur Alkylierung von Phenolen eingesetzt. Die dabei entstehenden Alkylaromaten sind Zwischenprodukte für die Herstellung von Detergenzien.

Diisobuten ist über die Dimerisierung, allgemein Oligomerisierung, von Isobuten erhältlich. Dabei entstehen neben den Dimeren (C₈) auch Oligomere höherer Molmassen (hauptsächlich C₁₂, C₁₆). Durch Gerüstumlagerungsreaktionen enthalten die C₈-Dimere neben dem Diisobuten noch geringe Anteile anderer C₈-Olefine. Setzt man als Edukt für die Oligomerisierung Isobuten in Mischungen mit anderen Olefinen ein, bilden sich zudem Cooligomere. In Gegenwart von 1-Buten entstehen beispielsweise 2,2-Dimethylhexene und 2,2,3-Trimethylpentene.

Für die wirtschaftliche Herstellung von Diisobuten, sei es als Edukt für Synthesen oder als Kraftstoffadditiv, aus Isobuten-haltigen Kohlenwasserstoffgemischen sind mehrere Kriterien zu beachten. Hierzu zählen eine hohe C₈-Selektivität, eine hohe C₈-Isomerenreinheit, die Langzeitstabilität des Katalysators, die technische Lösungen zur Abführung der anfallenden Reaktionswärme und bei Einsatz von Mischungen, die auch 1-Buten enthalten, eine geringe Isomerisierung des 1-Butens zu 2-Butenen.

Eine hohe C₈-Selektivität ist u.a. erwünscht, da die Tetramere des Isobutens (oder Cotetramere mit linearen C₄-Olefinen) oder ihre hydrierten Derivate für den Einsatz in Vergaserkraftstoffen nicht geeignet sind, da sie einen zu hohen Siedepunkt besitzen. Die Bildung von Tetrameren stellt daher bei der Herstellung von Kraftstoffadditiven einen realen Ausbeuteverlust dar. Die Siedepunkte der Trimeren bzw. ihrer hydrierten Derivate liegen mit 170-180°C im oberen Teil des Siedepunktbereichs für Vergaserkraftstoffe; sie können zwar anteilig in Kraftstoffen verwendet werden, dennoch ist ihre Bildung möglichst zu minimieren.

Eine hohe C₈-Isomerenreinheit, d.h. ein hoher Anteil an Diisobuten in der C₈-Fraktion und nur wenig Bildung von Codimeren (Dimer aus 1-Buten und Isobuten) und Umlagerungsprodukten, ist insbesondere bei der Herstellung von Diisobuten erwünscht, das Verwendung in Synthesen findet. Die Bildung von Codimeren mit beispielsweise n-Butenen ist zudem zu vermeiden, da dadurch n-Butene verbraucht werden, die sonst anderweitig (1-Buten beispielsweise als (Co)Monomer für Polymere) verwendet werden könnten.

Eine hohe Langzeitstabilität des Katalysators ist nicht nur erforderlich, um die Katalysatorkosten zu minimieren, sondern auch um den technischen Aufwand des Katalysatorwechsels gering zu halten.

Bei der Oligomerisierung von Isobuten wird zudem in erheblichen Mengen Wärme frei. Wird diese nicht in ausreichendem Maße abgeführt, heizt sich die Reaktionsmischung auf. Dies kann sowohl zur Verschlechterung der Selektivitäten führen als auch einen negativen Einfluss auf die Katalysatorstabilität haben.

Isobuten fällt technisch oft in Mischungen mit 1-Buten an. Destillativ sind die Stoffe mit technisch sinnvollem Aufwand nicht trennbar. Eine Trennung wird daher durch selektive chemische Umsetzung einer der beiden Komponenten erreicht, beispielsweise Verätherung des Isobutens. Bei der chemischen Umsetzung des Isobutens darf das 1-Buten sich aber nicht zu 2-Butenen umlagern (vgl. K.Weissermel, H.J. Arpe, Industrielle Organische Chemie, Wiley-VCH, 5. Auflage, 1998, Seite 74-82). 1-Buten ist ein begehrter Rohstoff, u.a. wird es als Comonomer bei der Herstellung von Polyolefinen eingesetzt.

Alle diese Kriterien sind bereits in der Literatur beschrieben und es wurden teilweise auch Lösungen gefunden. Ein Schwerpunkt der Arbeiten liegt bei der Entwicklung von Katalysatoren. Die Oligomerisierung von Isobuten kann durch Lewis-, Brönstedsäuren oder Koordinationsverbindungen, insbesondere denen des Nickels, katalysiert werden. Katalysatoren für diese Reaktion sind seit langem bekannt und Gegenstand zahlreicher Patente und Veröffentlichungen.

Die Oligomerisierung kann prinzipell homogen, d. h. unter Verwendung von im Reaktionsgemisch löslichen Katalysatoren, oder heterogen, d.h. unter Verwendung von im Reaktionsgemisch unlöslichen Katalysatoren durchgeführt werden. Der Nachteil der homogenen Verfahren besteht darin, dass der Katalysator den Reaktor mit den Reaktionsprodukten und nicht umgesetzten Edukten verlässt, von denen er abgetrennt, aufgearbeitet und entsorgt oder zurückgeführt werden muss.

Die meisten technischen Verfahren verwenden daher heterogene Katalysatoren, die zudem oft im Festbett angeordnet sind, so dass eine aufwendige Katalysatorabtrennung entfällt. Die meisten bekannten Festkatalysatoren gehören einer der folgenden Gruppen an:
a) Mineralsäuren (z. B. Schwefelsäure oder Phosphorsäure) auf einem Trägermaterial (z. B. Aluminiumoxid oder Siliciumdioxid)
b) Zeolithe oder andere Alumosilikate mit oder ohne Dotierung weiterer Metalle, insbesondere mit Übergangsmetallen
c) Saure Ionenaustauscherharze

Mineralsäuren auf Trägern sind als Katalysatoren nur wenig geeignet, da sie auch Gerüstumlagerungen (Reaktion von zwei Molekülen Isobuten zu anderen C₈-Isomeren als 2,4,4-Trimethylpenten) bewirken.

In EP 0 224 220 wird eine Buten-Oligomerisierung an einem mit Wismut und/oder Blei dotierten Zeolith-Katalysator durchgeführt. Dabei enthält die C₈-Fraktion über 4 % unerwünschte 2,3,4-Trimethylpentene. Zeolithe als Katalysatoren werden ebenfalls eingesetzt in US 4720600. Die Oligomerisierung von Isobuten an einem Röntgen-amorphen Alumosilikat wird in EP 0 536 839 offengelegt. Dabei kann selbst bei den milden Temperaturen von 60 - 65 °C ein Verlust an 2,2,4-Trimethylpentenen durch Skelettisomerisierung nicht vermieden werden. Die Isobuten-Oligomerisierung an einem Röntgen-amorphen Nickelalumosilikat wird in WO 93/06926 beschrieben. Dabei wird unverdünntes Isobuten bei 60 °C umgesetzt. Das Produktspektrum zeigt, dass die C8-Selektivität nicht besonders hoch ist. Bei einem Isobuten-Umsatz von 15 - 20% beträgt die C₈-Selektivität 85 - 86%, bei einem Umsatz von 75 % nur noch 72%. In US 3531539 wird Isobuten, das in Mischungen mit 1-Buten vorliegt, an einem Molekularsieb umgesetzt. US 3518323 offenbart die Umsetzung von Isobuten, das in Mischungen mit n-Butenen vorliegt, an einem geträgerten Nickel-Katalysator. Die selektive Dimerisierung von Isobuten aus Mischungen von C₄-Monoolefinen an heterogenen NickelKatalysatoren beschreibt auch US 3832418. In US 4197185 ist die Umsetzung von Isobuten aus Mischungen von C₄-Kohlenwasserstoffen an anorganischen Heterogenkatalysatoren Teil des beanspruchten Verfahrens.

Die Umsetzung von Isobuten an sauren Ionentauscherharzen ist seit langem bekannt und wurde auch in großtechnischen Prozessen genutzt (Hydrocarbon Prozessing, April 1973, Seite 171; Erdöl, Kohle, Erdgas, Petrochem. 1974, 27, Heft 5, Seite 240). Durch die Diskussion, die Verwendung von MTBE (Methyl-tert-butylether, wird technisch aus Isobuten und Methanol gewonnen) als Kraftstoffadditiv einzuschränken, finden Verfahren zur Dimerisierung von Isobuten wieder verstärkt Beachtung. Aus diesem Anlass wurde eine aktuelle Übersicht über Verfahrensvarianten zur Umsetzung von Isobuten an sauren Ionentauscherharzen publiziert; Hydrocarbon Processing, February 2002, Seite 81. Je nach Isobutengehalt des eingesetzten Rohstoffs kommen dabei unterschiedliche Verfahrensvarianten zum Einsatz, sowohl um die Exothermie der Reaktion zu beherrschen, als auch um die Selektivität zu steuern. Hauptsächlich wird die Zugabe von Moderatoren und/oder Verdünnung zur Herabsetzung der Isobutenkonzentration genutzt.
Der Einsatz von Moderatoren, die der Reaktion zugesetzt werden, um Aktivität und Selektivität des Katalysators zu steuern, ist Gegenstand diverser Patente. Typische Moderatoren sind beispielsweise Methyl-tert.-butylether, tert.-Butanol oder Wasser. Der prinzipielle Nachteil von Moderatoren besteht darin, dass der Moderator oder aus ihm entstandene Folgeprodukte vom Produkt abgetrennt werden müssen. Int US 4100220 wird der Einsatz von Wasser beziehungsweise TBA (tert.-Butylalkohol) als Moderator offenbart, WO 01/51435 beschreibt den Einsatz von TBA. US 4375576 und US 4447668 nutzen MTBE als Moderator, GB 2325237 Ether und primäre Alkohole, EP 1074534 tertiäre Alkohole, primäre Alkohole und Ether, EP 1074534 allgemein "Oxygenate". Da sich in Anwesenheit saurer Katalysatoren t-Butylether wie MTBE aus Isobuten und Alkoholen bildet, sind auch Verfahren zur parallelen Produktion von Ethern und Isobutenoligomeren bekannt (US 5723687).

Der Einsatz von Moderatoren bringt zudem Nachteile im technischen Betrieb mit sich. Als Ionentauscher eingesetzte Katalysatoren, die durch Zugabe von Moderatoren wie Wasser oder TBA in ihrer Aktivität gemindert werden, reagieren bei Lastwechseln des Reaktors sehr langsam. Nach Änderung der Zulaufmenge benötigt der Reaktor Zeit, um wieder in einen stationären Zustand zu gelangen. Ein stationäres Verhalten des Reaktors ist aber für einen einfachen und sicheren Betrieb der Anlage vorteilhaft. Ein Vorteil des Einsatzes von teilneutralisierten Ionentauschern liegt darin, dass die Zeit, die zum Erreichen eines stationären Reaktorverhaltens nach einem Lastwechsel benötigt wird, verkürzt wird.

Neben oder parallel zum Einsatz von Moderatoren wird die Verdünnung des Isobuten-haltigen Einsatzstoffes beschrieben, um bessere Selektivitäten, insbesondere aber auch Kontrolle der Exothermie zu erzielen (US 5877372, WO 01/51435, US 2002/0002316). US 5003124 beschreibt ein Verfahren, bei dem dem Problem der Abführung der Reaktionswärme begegnet wird, indem man nur teilweise in flüssiger Phase bei der Siedetemperatur der Flüssigphase arbeitet.

Die Schriften US 6 274 783 und WO 01/81278 haben beide Verfahren zur Oligomerisierung mit gleichzeitiger Hydrierung der Produkte zum Gegenstand.

In EP 0 417 407 werden Formkörper von stark sauren Ionenaustauschen als Katalysator für die Oligomerisierung von Isobuten eingesetzt. Diese Ionenaustauscher werden z. T. nachträglich mit Säure behandelt, um eine erhöhte Acidität zu erreichen. Die Ausbeute an Dimeren von 93 - 96 % ist gut. Allerdings wird die Zusammensetzung der C₈-Fraktion nicht offenbart.

In EP 0 048 893 wird ein Verfahren zur Herstellung von C₄-Oligomeren und Alkyl-tert.-butylethern beschrieben. Isobuten wird zusammen mit Alkoholen in flüssiger Phase an einem sauren Kationentauscher umgesetzt, der mit einem oder mehreren Metallen der Gruppe 7 oder 8 des Periodensystems belegt ist. Die Metalle liegen dabei in elementarer Form vor.

EP 0 325 144 offenbart die Verwendung von sauren Ionentauschern, die teilweise mit amphoteren Elementen (bevorzugt werden Aluminium, Chrom, Vanadium, Titan, Zirkon, Molybdän, Wolfram) beladen sind. Diese modifizierten Ionentauscher werden als Katalysatoren bei der Herstellung von tert.-Amylalkohol (TAA) aus i-Amylenen eingesetzt. Der Vorteil des Verfahren liegt in einem erhöhten Umsatz der i-Amylene und gleichzeitger Unterdrückung der Oligomerisierung der i-Amylene.

Es besteht daher ein Bedarf an der effizienten Herstellung von Diisobuten, bzw. der Abtrennung von Isobuten zur Herstellung von 1-Buten aus C₄-Kohlenwasserstoffgemischen.

Es wurde überraschend gefunden, dass diese Aufgabe durch Oligomerisierung von Isobuten in flüssiger Phase an einem saurem Ionenaustauscherharz mit Sulfonsäurengruppen gelöst werden kann, wenn ein Teil der Protonen des sauren Ionentauschers gegen Metallionen ausgetauscht sind.

Gegenstand der vorliegenden Erfindung ist daher ein Verfahren zur Oligomerisierung von Isobuten in Gegenwart von n-Butenen an einem festen, sauren Ionenaustauscher, wobei die Umsetzung an einem Ionenaustauscher durchgeführt wird, dessen acide Protonen teilweise gegen Metallionen ausgetauscht wurden.

Diese Technik kann auch zur effizienten Gewinnung von 1-Buten eingesetzt werden. Ein weiterer Gegenstand der Erfindung ist daher ein Verfahren zur Herstellung von 1-Buten gemäß Anspruch 11.

Saure Ionenaustauscherharze sind für die Oligomerisierung von Isobuten nur dann brauchbare Katalysatoren, wenn sie eine gewisse Mindestacidität aufweisen. So sind Harze mit Carbonsäuregruppen häufig nicht acide genug und daher als Katalysator in der Regel nicht geeignet. Geeignete Harze sind jene mit Sulfonsäuregruppen. Ihre Aktivität in der Oligomerisierung von Isobuten kann durch Moderatoren wie Alkohole (TBA), Ether (MTBE) oder Wasser herabgesetzt werden.

Aus der Literatur ist bekannt, die Säurestärke von Ionenaustauschern mit Sulfonsäuregruppen durch teilweisen Ionenaustausch herabzusetzen (Structure-breaking Effect of Metal Ions influencing the Acidity of an Anhydrous Acid, C. Buttersack, H. Widdecke, J. Klein, Journal of Molecular Catalysis, 40 (1987) 23 -25). Es ist jedoch überraschend, dass ein derart modifiziertes Ionenaustauscherharz vorteilhaft bei der Darstellung von Kraftstoffadditiven aus Isobuten-haltigen Kohlenwasserstoffmischungen eingesetzt werden kann.

In den erfindungsgemäßen Verfahren werden feste sulfonierte Ionenaustauscherharze eingesetzt, bei denen insbesondere 0.1 bis 60 %, 0.1 bis 50 %, 0.1 bis 40 %, 0.1 bis 30 %, 0.1 bis 29 %, bevorzugt 0.5 bis 20 %, besonders bevorzugt 5 bis 15 % der aciden Protonen der Sulfonsäuregruppen gegen Metallionen ausgetauscht worden sind. Als Metallionen, die die Protonen ersetzen, können Alkalimetall-, Erdalkalimetall-, Chrom-, Mangan-, Eisen, Kobalt-, Nickel, Zink- und Aluminiumionen sowie Ionen der Lanthanidgruppe (Seltene Erden) verwendet werden. Bevorzugt werden dafür Alkalimetallionen, insbesondere Natriumionen eingesetzt. Es ist auch möglich, dass das Harz mit zwei oder mehreren unterschiedlichen Metallionen beladen ist.

Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfongruppen verwendet. Die Eigenschaften dieser Harze, insbesondere spezifische Oberfläche, Porosität, Stabilität, Quellung bzw. Schrumpfung und Austauschkapazität, können durch den Herstellprozess variiert werden. Die Harze können gelförmig, makroporös oder schwammförmig hergestellt werden. Stark saure Harze des Styrol-Divinylbenzol-Typs werden u. a. unter folgenden Handelsnamen verkauft: CT 151 der Firma Purolite, Amberlyst 15, Amberlyst 35, Amberlite IR-120, Amberlite 200 der Firma Rohm&Haas, Dowex M-31 der Firma DOW, K 2611, K 2431 der Firma Bayer.
Die Ionenaustauscherkapazität der vollständig in der H⁺-Form vorliegenden Harze liegt typischer Weise zwischen 1 bis 2, insbesondere 1,5 bis 1,9 Mol H⁺ pro Liter feuchtes Harz (handelsüblich).

Im Verfahren der Erfindung werden vorzugsweise macroporöse Harze eingesetzt, wie beispielsweise K 2431 der Firma Bayer, Amberlyst 15 oder Amberlyst 35 der Firma Rohm & Haas. Das Porenvolumen beträgt bevorzugt 30 bis 60 ml/g, insbesondere 40 bis 50 ml/g (bezogen auf handelsübliches wasserfeuchtes Harz).

Die Korngröße des Harzes liegt bevorzugt zwischen 500 µm und 1500 µm, insbesondere zwischen 600 µm und 1000 µm.

Die Korngrößenverteilung kann enger oder weiter gewählt werden. So können beispielsweise Ionenaustauscherharze mit sehr einheitlicher Korngröße (monodisperse Harze) eingesetzt werden.

Es kann vorteilhaft sein, in Reaktoren, die mit hohen Lineargeschwindigkeiten durchströmt werden, zur Verringerung des Differenzdruckes ein größeres Korn, und in Reaktoren, die mit einer geringen Lineargeschwindigkeit durchströmt werden, zur Erzielung des optimalen Umsatzes ein kleineres Korn einzusetzen.

Optional können die Ionenaustauscherharze als Formkörper, wie beispielsweise Zylinder, Ringe oder Kugeln, eingesetzt werden.

Bei der Verwendung mehrerer Reaktoren können diese mit Harz gleicher oder unterschiedlicher Korngröße (bzw. Korngrößenverteilung) gefüllt sein.

Für die Herstellung der teilneutralisierten Ionenaustauscherharze können verschiedene Verfahren, die alle in der Fachliteratur beschrieben werden, angewendet werden. Liegt das Ionenaustauscherharz in der H-Form vor, können Protonen gegen Metallionen ausgetauscht werden. Liegt das Harz als Metallsalz vor, können Metallionen mit Hilfe von Säuren durch Protonen ersetzt werden. Prinzipiell kann dieser Ionenaustausch sowohl in organischer als in wässriger Suspension erfolgen.
In einem einfachen Verfahren wird beispielsweise das Ionentauscherharz in der H⁺-Form mit so viel Flüssigkeit aufgeschlämmt, dass eine gut rührbare Suspension entsteht. Dazu wird eine Lösung, die die gewünschten Ionen enthält, zudosiert. Nach erfolgten Ionenaustausch wird das teilausgetauschte Ionenaustauscherharz gewaschen und getrocknet.

Die Lösungsmittelmenge zum Aufschlämmen des Ionenaustauscherharz liegt typischerweise beim ein- bis zehnfachen des Eigenvolumens des Ionenaustauscherharzes. Für die Herstellung der Lösung mit der gewünschten Ionenart, die zudosiert wird, ist es ratsam, ein Lösungsmittel zu wählen, das mit dem Lösungsmittel, in dem das Harz suspendiert ist, mischbar ist. Es ist zweckmäßig, gleiche Lösungsmittel zu verwenden.
Der Ionenaustausch erfolgt vorzugsweise im Temperaturbereich von 10 bis 100 °C, besonders bevorzugt bei 20 bis 40 °C. Der Austausch ist in der Regel nach spätestens 24 Stunden abgeschlossen. Nach dem Ionenaustausch wird der Katalysator von der Lösung getrennt, z. B. durch Dekantieren oder Filtrieren, und optional anschließend mit einem Lösungsmittel gewaschen. Es ist zweckmäßig das gleiche Lösungsmittel zu verwenden, in dem der Katalysator suspendiert war.

Es ist vorteilhaft, den feuchten Katalysator zu getrocknen, einerseits um ihn handhabbarer (rieselfähiger) zu machen und um andererseits in den ersten Tagen nach dem Anfahren des Reaktors die Verunreinigung des Produktes durch das anhaftende Lösungsmittel oder dessen Folgeprodukte gering zu halten. Die Trocknung kann im Vakuum oder im Inertgasstrom, beispielsweise im Stickstoffstrom, erfolgen. Die Trockentemperaturen liegen typischerweise zwischen 10 und 120 °C.

Ein bevorzugter Weg zur Herstellung der im erfindungsgemäßen Verfahren verwendeten Katalysatoren ist der Austausch von Protonen gegen Metallionen in wässriger Phase, Waschen des teilausgetauschten Ionentauscherharzes mit Wasser und anschließende Trocknung

Die Ionen, mit denen das Harz beladen werden soll, können als Lösungen von Hydroxiden, oder Salzen organischer oder anorganischer Säuren vorliegen. Bei Salzen mehrbasiger Säuren können auch saure Salze eingesetzt werden. Ebenfalls können Verbindungen mit anderen organischen Resten wie beispielsweise Alkoholate oder Acetylacetonate verwendet werden. Als Quelle für die Metallionen werden bevorzugt Metallhydroxide und Salze anorganischer Säuren eingesetzt. Ganz besonders bevorzugt ist der Einsatz der Alkalimetallhydroxide (zum Beispiel Natriumhydroxid), Alkalimetallhalogenide (zum Beispiel Natriumchlorid), Alkalimetallsulfate (zum Beispiel Natriumsulfat), Alkalimetallnitrate (zum Beispiel Natriumnitrat), Erdalkalimetallhydroxide und Erdalkalimetallnitrate.

Nach dem oben beschriebenen Vorgehen können je nach Austauschgrad, Ionenart und Harz Katalysatoren unterschiedlicher Aktivität und Selektivität hergestellt werden.

Ein Reaktor in dem erfindungsgemäßen Verfahren kann ein Gemisch von Harzen unterschiedlicher Reaktivität enthalten. Ebenso ist es möglich, dass ein Reaktor Katalysatoren mit unterschiedlicher Aktivität, in Schichten angeordnet, enthält. Wird mehr als ein Reaktor verwendet, können die einzelnen Reaktoren mit Katalysatoren gleicher oder unterschiedlicher Aktivität(en) gefüllt sein.

Als Ausgangsstoffe werden Isobuten-haltige Kohlenwasserstoffgemische eingesetzt. Bevorzugt werden Mischungen von Isobuten mit anderen C₄-Kohlenwasserstoffen (Anteil an C₄-Komponenten größer 95 %) eingesetzt.

Technische Gemische, die Isobuten enthalten, sind beispielsweise Leichtbenzinfraktionen aus Raffinerien, C₄-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind allesamt in der Fachliteratur beschrieben, K.Weissermel, H.J. Arpe, Industrielle Organische Chemie, Wiley-VCH, 5. Auflage, 1998, Seite 23-24; 65-99; 122-124.

Bevorzugt eingesetzt werden C₄-Fraktionen aus Steamcrackern (die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden) oder Katcrackern. Die als Nebenprodukt anfallenden C₄-Schnitte enthalten je nach Crack-Verfahren unterschiedliche Mengen an Isobuten. Weitere Hauptbestandteile sind 1,3-Butadien, 1-Buten, c-2-Buten, t-2-Buten, n-Butan und i-Butan. Typische Isobuten-Gehalte in der C₄ Fraktion liegen bei C₄-Fraktionen aus Steam-Crackern bei 18 bis 35%, bei FCC-Katcrackern bei 10-20 %.

Für das erfindungsgemäße Verfahren ist es vorteilhaft, mehrfach ungesättigte Kohlenwasserstoffe wie 1,3-Butadien aus dem Einsatzgemisch zu entfernen. Dies kann nach bekannten Verfahren, beispielsweise durch Extraktion, Extraktivdestillation oder Komplexbildung erfolgen (vgl. K.Weissermel, H.J. Arpe, Industrielle Organische Chemie, Wiley-VCH, 5. Auflage, 1998, Seite 119-121)
Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. beschrieben in EP 52 3482. Auch durch selektive Umsetzungen des 1,3-Butadiens, zum Beispiel Dimerisierung zum Cyclooctadien, Trimerisierung zum Cyclododecadien, Polymerisations- oder Telomerisationsreaktionen, kann das 1,3-Butadien zumindest teilweise entfernt werden. Wurde ein Crack C₄-Schnitt als Rohstoff eingesetzt, bleibt in allen Fällen ein Kohlenwasserstoffgemisch (Raffinat I oder hydriertes Crack-C₄ (HCC4)) zurück, das hauptsächlich die gesättigten Kohlenwasserstoffe, n-Butan und Isobutan und die Olefine Isobuten, 1-Buten und 2-Butene enthält.

Die in dem erfindungsgemäßen Verfahren eingesetzten Isobuten-haltigen Kohlenwasserstoffmischungen können die folgenden Zusammensetzungen aufweisen:

| **Typische Zusammensetzung von selektiv hydriertem CC4 und Raffinat I, die aus dem C4-Schritt eines Stream-Cracker (High Severity) gewonnen werden** | | | | |
|---|---|---|---|---|
| | HCC4 | | Raffinat I | |
| Komponente | min [%] | max [%] | min [%] | max [%] |
| Isobutan | 1 | 4.5 | 1.5 | 8.0 |
| n-Butan | 5 | 8 | 6.0 | 15.0 |
| t-Buten | 18 | 21 | 7.0 | 10. |
| 1-Buten | 35 | 45 | 15.0 | 35.0 |
| Isobuten | 22 | 28 | 33.0 | 50.0 |
| c-Buten | 5 | 8 | 4.0 | 7.0 |
| 1,2-Butadien | | | | < 1% |
| 1,3-Butadien | 500 ppm | 2000 ppm | | < 1% |
| Monovinylacetylen | | | | < 1% |

| **Typische Zusammensetzung von Raffinat I, das aus C4-Schritten von Katcrackern und Steam-Cracker (High Severity) gewonnen wird** | | |
|---|---|---|
| | Raff. I Basis Katcracker | Raff. I Basis Steamcracker (Low Severity) |
| Komponente | typisch [%] | typisch [%] |
| Isobutan | 37 | 3 |
| n-Butan | 13 | 6 |
| t-Buten | 12 | 10 |
| 1-Buten | 12 | 27 |
| Isobuten | 15 | 44 |
| c-Buten | 11 | 10 |
| 1,3-Butadien | << 1 % | << 1 % |

Das Raffinat I bzw. HCC4 ist, neben anderen, ein bevorzugte eingesetztes Isobuten-haltiges Kohlenwasserstoffgemisch im Rahmen dieser Erfindung. Da Anlagen zur Aufarbeitung von C₄-Kohlenwasserstoffen in der Regel als Strang (Verbund mehrerer Anlagen) aufgebaut sind, ist es jedoch möglich, dass das Raffinat I bzw. HCC4 vor dem Eintritt in das erfindungsgemäße Verfahren eine oder mehrere andere Prozessstufen durchläuft. Auf diese Weise kann jeweils ein individuell angepasstes Gesamtkonzept zur Aufarbeitung mit dem entsprechenden Produktportfolio realisiert werden.

Typische Prozessstufen, die den erfindungsgemäßen Verfahren vorgelagert sein können, sind Wasserwäsche, Reinigung an Adsorbern, Selektivhydrierung, TBA-Synthese und andere selektive Umsetzungen, Trocknung, Hydroisomerisierung und Destillation.

### Wasserwäsche

Durch eine Wasserwäsche können hydrophile Komponenten aus dem Isobuten-haltigen Kohlenwasserstoffgemisch ganz oder teilweise entfernt werden, beispielsweise Stickstoffkomponenten. Beispiele für Stickstoffkomponenten sind Acetonitil oder N-Methylpyrrolidon (die z.B. aus einer 1,3-Butadien-Extraktivdestilltion stammen können). Auch Sauerstoffverbindungen (z. B. Aceton aus FCC-Cracker) können zum Teil über eine Wasserwäsche entfernt werden. Der Isobuten-haltige-Kohlenwasserstoffstrom ist nach einer Wasserwäsche mit Wasser gesättigt. Um eine Zweiphasigkeit im Reaktor zu vermeiden, sollte die Reaktionstemperatur um ca. 10 °C über der Temperatur der Wasserwäsche liegen.

### Adsorber

Adsorber werden eingesetzt, um Verunreinigungen zu entfernen. Dies kann beispielsweise vorteilhaft sein, wenn in einem der Prozessschritte Edelmetallkatalysatoren zum Einsatz kommen. Oftmals werden Stickstoff oder Schwefelverbindungen über vorgeschaltete Adsorber entfernt. Beispiele für Adsorber sind Aluminiumoxide, Molekularsiebe, Zeolithe, Aktivkohle, mit Metallen imprägnierte Tonerden. Adsorber werden von diversen Firmen vertrieben, beispielsweise der Firma Alcoa (Selexsorb®).

### Selektivhydrierung

Noch in geringen Mengen enthaltene mehrfach ungesättigte Verbindungen, insbesondere 1,3-Butadien, können gegebenenfalls durch weitere selektive Hydrierungsschritte (vergleiche EP 081041; Erdöl, Kohle, Erdgas, Petrochem. 1986, 39, 73) weiter verringert werden. (Pentanal, 2-Methylbutanal) können destillativ abgetrennt werden.

### TBA-Synthese

Teile des Isobutens können mit Wasser zu tert.-Butanol (TBA) umgesetzt werden. Verfahren zur Herstellung von TBA aus Isobuten-haltigen Kohlenwasserstoffmischungen sind Stand der Technik (vergleiche zum Beispiel Erdöl, Erdgas, Kohle, 1987, 103, 486). TBA wird beispielsweise als Lösungsmittel eingesetzt, wird aber auch durch Rückspaltung zu Isobuten und Wasser zur Darstellung von hochreinem Isobuten verwendet.

### Trocknung

Im Isobuten-haltigen Kohlenwasserstoffgemisch gegebenenfalls enthaltenes Wasser, das beispielsweise aus der Wasserwäsche oder der TBA-Synthese stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen C₄-Kohlenwasserstoffen ausgenutzt werden oder es werden Schleppmittel zugesetzt. Der Wassergehalt des Isobuten-haltigen Kohlenwasserstoffgemischs sollte nach der Trocknung ca. 10 - 50 ppm betragen.

### Hydroisomerisierung

Mit dem Verfahren der Hydroisomerisierung kann die Lage von Doppelbindungen im Molekül verschoben werden. Klassisches Beispiel ist die Hydroisomerisierung von 1-Buten zu 2-Butenen. Gleichzeitig werden mehrfach ungesättigte Verbindungen (beispielsweise Reste an 1,3-Butadien) zu einfachen Olefinen hydriert. Hydroisomerisierungsverfahren sind Stand der Technik und beispielsweise Teil der Patentanmeldungen GB 2057006, US 3764633 und DE 2223513.

Wird durch eine dem erfindungsgemäßen Verfahren vorgeschaltete Hydroisomerisierung das Verhältnis der Anteile 1-Buten / 2-Butene im Feed verschoben, so hat dies Auswirkungen auf die Bildung der Codimere, die im erfindungsgemäßen Verfahren entstehen. So ist es beispielsweise möglich, aus Isobuten-haltigen Kohlenwasserstoffmischungen, in denen der 1-Butengehalt durch Hydroisomerisierungsverfahren bis nahe an das thermodynamische Gleichgewicht reduziert wurde, durch Dimerisierung an teilneutralisierten Ionentauschern Produkte zu erzeugen, in denen 2,2-Dimethylhexene (Codimere aus Isobuten und 1-Buten) in beispielsweise möglich, aus Isobuten-haltigen Kohlenwasserstoffmischungen, in denen der 1-Butengehalt durch Hydroisomerisierungsverfahren bis nahe an das thermodynamische Gleichgewicht reduziert wurde, durch Dimerisierung an teilneutralisierten Ionentauschern Produkte zu erzeugen, in denen 2,2-Dimethylhexene (Codimere aus Isobuten und 1-Buten) in unter 1 % Anteil enthalten sind. Aus derartigen Mischungen kann hochreines Diisobuten mit einer Reinheit von >98.5% destillativ abgetrennt werden.

Die Hydroisomerisierung hat insbesondere dadurch Bedeutung erlangt, dass bei einer weitgehenden Isomerisierung des 1-Butens zum 2-Buten das Isobuten mit den Resten an 1-Buten und ggf. zusammen mit Isobutan destillativ von den restlichen C₄-Kohlenwasserstoffen abtrennbar ist. Durch geschickte Kombination von Destillation und Hydroisomerisierung, beispielsweise in einer Reaktivdestillation, kann man auf diese Weise Isobuten-haltige Ströme erzeugen, die weitgehend frei von n-Butenen sind. Auch diese Ströme eignen sich als Rohstoff für das erfindungsgemäße Verfahren. (EP 1 184 361 beschreibt den Einsatz eines auf diese Art erzeugten Rohstoffs für die Oligomerisierung von Isobuten, wobei die Oligomerisierug als Reaktivdestillation ausgeführt ist). Das Produkt der Oligomerisierung am teilneutralisierten Ionentauscher ist dann weitgehend frei von C₈-Codimeren.

### Destillation

Die Isobuten-haltigen Kohlenwasserstoffmischungen können durch Destillation in Fraktionen mit unterschiedlichen Isobutenkonzentrationen zerlegt werden. Dies kann sowohl direkt mit dem Raffinat I bzw. dem HCC4 erfolgen oder nachdem eine oder mehrere andere Prozessstufen durchlaufen wurden (vergleiche Hydroisomerisierung). Durch direkte Destillation des Raffinats I bzw. des HCC4s ist beispielsweise eine Trennung in eine an 2-Butenen und n-Butan verarmte, Isobuten reichere Fraktion gewinnbar.

### Zur erfindungsgemäßen Oligomerisation

Für die technische Ausführung der Umsetzung der Isobuten-haltigen Kohlenwasserstoffmischungen sind diverse Varianten möglich. Die Umsetzung kann chargenweise oder bevorzugt in kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich meistens, jedoch nicht ausschließlich, eines Festbetts. Eine anderes Konzept als Festbettreaktoren sind beispielsweise Reaktoren, in denen der Ionentauscher suspendiert in einer flüssigen Phase vorliegt (vergleiche "Bayer Verfahren", Erdöl und Kohle, Erdgas, Petrochemie, 1974, 27, Heft 5, Seite 240).
Wenn ein Festbett-Strömungsreaktor verwendet wird, kann die Flüssigkeit aufwärts oder abwärts strömen. Meistens wird ein Abwärtsströmen der Flüssigkeit bevorzugt. Eine den Reaktor umströmende Kühlflüssigkeit kann gegebenenfalls gleich oder entgegengesetzte Strömungsrichtung aufweisen. Weiterhin ist es möglich, den Reaktor unter Produktrückführung oder im geraden Durchgang zu betreiben.

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden.

Die Umsetzung der Isobuten-haltigen Kohlenwasserstoffmischungen an sauren Ionentauschern verläuft unter Freisetzung von Energie, was zu einer Erwärmung der Reaktionsmischung führt. Zur Begrenzung des Temperaturanstiegs sind bei Einsatz von nicht teilneutralisierten Ionentauschern oftmals Moderatoren notwendig, die die Aktivität des Katalysators herabsetzen. Als Moderatoren werden beispielsweise Alkohole (TBA), Ether (MTBE) oder Wasser eingesetzt. Ein Nachteil des Einsatzes von Moderatoren ist, dass man sie vom Produktstrom abtrennen muss (vgl. Hydrocarbon Processing, February 2002, page 81). Nur wenn sie in geringer Konzentration enthalten sind, können sie gegebenenfalls im Produkt verbleiben.

Ein weiteres probates Mittel zur Begrenzung des Temperaturanstiegs ist die Verdünnung der Isobuten-haltigen Kohlenwasserstoffmischungen, beispielsweise durch Rückführung von Produkt oder durch Zugabe inerter Kohlenwasserstoffe, beispielsweise von Diisobutan.
Durch die durch partielle Neutralisation herabgesetzte Katalysatoraktivität sind die Reaktionsgeschwindigkeiten pro Katalysatorvolumen geringer als beim Einsatz von nicht ausgetauschten Ionenaustauscherharzen. Mit dem geringeren Umsatz ist auch die entstehende Wärmemenge geringer. Dadurch kann bei entsprechendem Reaktorkonzept die Wärme in ausreichendem Maß abgeführt und auf den Einsatz von Moderatoren verzichtet werden. In einer bevorzugten Ausführungsform der erfindungsgemäßen Verfahren werden daher keine Moderatoren zugesetzt.

Die im technischen Prozess eingesetzten Reaktoren können adiabatisch, polytrop oder praktisch isotherm betrieben werden. Praktisch isotherm bedeutet, dass die Temperatur an einer beliebigen Stelle im Reaktor maximal um 10°C höher ist als die Temperatur am Reaktoreingang. Bei adiabatischem Betrieb der Reaktoren ist es in der Regel sinnvoll, mehrere Reaktoren in Reihe zu schalten und zwischen den Reaktoren zu kühlen. Reaktoren, die für einen polytropen oder praktisch isothermen Betrieb geeignet sind, sind beispielsweise Rohrbündelreaktoren, Rührkessel und Schlaufenreaktoren.
Der Reaktor kann auch in Form einer Reaktivdestillation betrieben werden, die katalytische Packungen mit teilneutralisierten Ionentauschern enthält. Als Beispiele für katalytische Packungen seien Katapack® der Firma Sulzer und Multipack® der Montz GmbH angeführt.

Es ist möglich, mehrere Reaktoren, auch verschiedener Bauarten, zu kombinieren. Dabei kann nach jedem Reaktor optional eine destillative Abtrennung von Reaktionsprodukten erfolgen. Es ist zudem möglich, Reaktoren unter Rückführung von Produkt zu betreiben.

Die Temperaturen, bei denen die Oligomerisation betrieben werden, liegen zwischen 5 und 160 °C, vorzugsweise zwischen 40 und 110 °C.

Die Umsetzung kann mit und ohne Zugabe eines zusätzlichen Lösungsmittels erfolgen. Als Lösungsmittel werden bevorzugt gesättigte Kohlenwasserstoffe eingesetzt, insbesondere C₄-, C8- oder C₁₂ Kohlenwasserstoffe. Ganz besonders bevorzugt ist der Einsatz von Isooktan. Bei Zugabe von Lösungsmitteln beträgt ihr Anteil 0 bis 60 Gew.%, bevorzugt 0 bis 30 Gew.%.

Die erfindungsgemäße Umsetzung kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 40 bar, d. h. die Isobuten-haltigen Kohlenwasserstoffmischungen liegen während der Oligomerisation ganz oder teilweise in flüssiger Phase vor. Wenn die Reaktion vollständig in der Flüssigphase durchgeführt werden soll, sollte der Druck 2 bis 4 bar höher als der Dampfdruck des Reaktionsgemisches sein, um in den Reaktoren Verdampfungsprobleme zu vermeiden.

Auch wenn die Reaktion bei einem Druck betrieben, bei dem die Reaktionsmischung nicht vollständig flüssig vorliegt (beispielsweise in einer Reaktivdestilation oder bei Verfahrensvarianten analog US 5003124), findet die Oligomerisierung nach dem erfindungsgemäßen Verfahren trotzdem in der Flüssigphase, also an "feuchtern" d. h. mit Flüssigkeit benetztem Katalysator statt.

Der Gesamtumsatz an Isobuten kann über die Art und Menge des verwendeten Katalysators, den eingestellten Reaktionsbedingungen und Anzahl der Reaktionsstufen eingestellt werden. Prinzipiell ist es möglich, das Isobuten in den Isobuten-haltigen Kohlenwasserstoffmischungen nahezu quantitativ umzusetzen. Bei Anwesenheit anderer Olefine, also beispielsweise bei Einsatz von Raffinat I als Rohstoff, kann die Bildung von Codimeren von Isobuten mit anderen Olefinen stark zunehmen. Daher ist es vorteilhaft, den Umsatz des Isobutens auf 95 %, besser noch auf 90% zu begrenzen.

Der Austrag der erfindungsgemäßen Oligomerisierung kann auf unterschiedliche Weise aufgearbeitet werden. Bevorzugt wird der Austrag der Oligomerisation in C₈-Olefine, d. h. hauptsächlich Diisobuten und C₄-Olefine, d. h. nicht umgesetztes Olefin wie Isobuten, 1-Buten und/oder 2-Buten und die inerten Butane getrennt.

Eine bevorzugte Form der Trennung dieser Fraktionen ist die destillative Trennung. Dabei wird zunächst nicht umgesetztes Isobuten zusammen mit weiteren Leichtsiedern (Fraktion (A), Kohlenwasserstoffe mit weniger als 7 Kohlenstoffatomen) von den Oligomeren abgetrennt.
Wird bei dem erfindungsgemäßen Verfahren Raffinat I als Rohstoff eingesetzt, dann erhält man bei der Destillation als Fraktion (A) eine Mischung, die in ihrer Zusammensetzung dem nahe kommt, was häufig als Raffinat II bezeichnet wird. Reste an nicht umgesetzten Isobuten können, falls erwünscht, aus dieser Mischung nach bekannten Verfahren entfernt werden, beispielsweise durch Verätherung mit Alkoholen (DE 2853769; Ullmann's Encyclopedia, Sixth Edition, Electronic Release, Methyl tert-Butyl Ether - Produktion).

Das in der C₄-Olefin-haltigen Fraktion noch vorhandene Isobuten kann optional in mindestens einer weiteren Reaktionsstufe mit einem Alkohol verethert werden. Bevorzugt wird hierzu Methanol oder Ethanol unter Erhalt von MTBE oder ETBE verwendet.

Die Umsetzung des restlichen Isobutens mit Alkoholen zu Ethern kann auch vor der destillativen Trennung erfolgen. Ein entsprechendes Verfahren wird in DE 2944457 beschrieben. Der Austrag der Oligomerisierung wird mit Alkoholen, vorzugsweise Methanol, an sauren Katalysatoren umgesetzt. Dabei bildet sich aus dem noch enthaltenen Isobuten ein tert.-Butylether. Aus dieser Mischung kann dann destillativ beispielsweise ein Raffinat II, welches ggf. noch Mengen an Alkohol enthält, abgetrennt und weiter verarbeitet werden.
Aus Raffinat II, welches nur noch geringe Mengen an Isobuten aufweist, kann 1-Buten von hoher Reinheit gewonnen werden (Ullmann's Encyclopedia, Sixth Edition, Electronic Release, Butenes - Upgrading of Butenes). Andere Verwendungsmöglichkeiten für Raffinat II sind die Oligomerisierung der enthaltenen Butene, beispielsweise an heterogenen Nickelkatalysatoren. Dabei wird unter anderem Di-n-buten erhalten, aus dem nach Hydroformylierung, Hydrierung und Veresterung Weichmacher (z.B. Diisononylphthalat) für Kunststoffe hergestellt werden.

Nach der Abtrennung von Isobuten zusammen mit weiteren Leichtsiedern (Fraktion (A), Kohlenwasserstoffe mit weniger als 7 Kohlenstoffatomen) erhält man eine hauptsächlich C₈-Kohlenwasserstoffe enthaltende Fraktion. Diese enthält neben dem Diisobuten auch die Codimeren und höhere Oligomere (C₁₂, C₁₆). Diese Fraktion kann in weiteren Destillationsschritten weiter fraktioniert werden. So ist es beispielsweise möglich, eine Fraktion mit hochreinem Diisobuten abzutrennen, um diese separat, beispielsweise für chemische Synthesen, einzusetzen. Für den Einsatz las Vergaserkraftstoffkomponente kann es notwenig sein, hochsiedende Komponenten (vorzugsweise Siedepunkt > 220 °C) abzutrennen.

Es ist auch möglich die gesammte, die C₈-Olefine enthaltende Fraktion ganz oder teilweise zu den gesättigten Kohlenwasserstoffen zu hydrieren.

Wird vor der Abtrennung der Leichtsieder eine Verätherung des noch enthaltenen Isobutens vorgenommen, dann enthält die Fraktion mit den Oligomeren des Isobutens neben den Oligomeren noch Ether und Reste an Alkohol. Diese Mischung kann destillativ nach bekannten Verfahren in einzelne Fraktionen (die beispielsweise als Hauptbestandteile Ether oder Alkohol oder Oligomere enthalten) aufgetrennt werden. Eine oder mehrere dieser Fraktionen (Cₙ) können beispielsweise als Kraftstoffzusatz eingesetzt werden.

Für den Einsatz als Vergaserkraftstoffkomponente kann es vorteilhaft sein, die olefinischen Doppelbindungen der Oligomere ganz oder teilweise zu hydrieren.
Methoden zur Hydrierung der Produkte der Oligomerisierung zu den entsprechenden Parafinen sind dem Fachmann hinlänglich bekannt. Gängige Methoden zur Hydrierung von Olefinen sind beispielsweise beschrieben in F.Asinger,. "CHEMIE UND TECHNOLOGIE DER MONOOLEFINE", AKADAMIE VERLAG, BERLIN, 1957, Seite 626 - 628 oder DE 197 19 833.

In einer bevorzugten Ausführungsform wird die Hydrierung in flüssiger Phase an einen heterogenen Hydrierkatalysator durchgeführt. Als Hydrierkatalysatoren werden bevorzugt Pt, Pd und Ni auf anorganischen Trägermaterialien verwendet. Die Temperatur, bei der die Hydrierung durchgeführt wird, liegt bevorzugt im Bereich von 10 bis 250°C, der Druck zwischen 1 bar und 100 bar.

Nach der Hydrierung können durch destillative Trennung weitere Fraktionen gewonnen werden. Aus diesen und aus unhydrierten Fraktionen sind durch Abmischung Kraftstoffadditive bestimmter Eigenschaften erhältlich.

Die folgenden Beispiele sollen die Erfindung weiter erläutern, nicht aber ihren Anwendungsbereich beschränken, der sich aus den Patentansprüchen ergibt.

### Beispiele

### Beispiel 1

### Herstellung eines teilneutralisierten Katalysators, Einstellung der Säurekapazität

Der eingesetzte Ionenaustauscher der Fa. Rohm und Haas (Amberlyst 15) hatte eine ursprüngliche Säurekapazität von 1,43 mol H+/1. Zur Einstellung der gewünschten Aktivität wurden 50 % der sauren Zentren neutralisiert.

Hierfür wurden 1000 ml des Ionenaustauscherharzes in 1000 ml VE-Wasser aufgeschlämmt und unter starkem Rühren eine Lösung aus 28,6g Natriumhydroxid (0,715 mol) und 500 ml VE-Wasser in einer Stunde im Temperaturbereich 20 bis 40 °C zugetropft. Es wurde 5 min nachgerührt und danach das Ionenaustauscherharz mit dreimal 1000 ml VE-Wasser neutral gewaschen. Die anschließende Kapazitätsmessung des teilneutralisierten Ionenaustauschers ergab 0,715 +/- 0,03 mol H+/1. Der Katalysator wurde für 15 h bei 70 °C getrocknet.

Die Herstellung anderer teilneutralisierter Ionenaustauscherharze erfolgt analog zu Beispiel 1, indem das saure Ionenaustauscherharz, suspendiert in Wasser, mit der berechneten Menge einer wässrigen Alkalihydroxid-Lösung umgesetzt wird.

Die Oligomerisierungsversuche (Beispiele 2 bis 6) wurden in einem ummantelten Laborrohrreaktor von 2 m Länge und 6 mm inneren Durchmesser durchgeführt. Mit Hilfe eines Wärmeträgeröls, das durch den Reaktormantel gepumpt wurde, konnte der Reaktor temperiert werden. Bei allen Versuchen wurde ein Isobutan-Isobuten-Gemisch bei 22 bar oligomerisiert.

### Beispiele 2 bis 4

Bei den Versuchen 2-4 wurden jeweils 54 ml eines (getrockneten) mit Na⁺-teilneutralisierten Ionentauschers des Typs Amberlyst 15 eingesetzt. Der Grad des Austausches der Protonen wurde jeweils durch eine Bestimmung der Kapazität des Ionentauschers überprüft.
Die Oligomerisierung des Isobutens erfolgte jeweils in einem Rohreaktor von 200 cm Länge (zu einer Spirale gewickelt), Innendurchmesser 6 mm, in den der Katalysator eingefüllt wurde. Das Rohr wird von außen durch ein Ölbad temperiert. Der Rohstoff wird über eine Pumpe durch den Reaktor gefördert; am Ausgang des Reaktors wird der Druck über eine Druckhaltung auf konstant 22 bar eingeregelt.
Die Betriebsbedingungen der einzelnen Versuche, Analysen von Zulauf und Austrag sind in der nachfolgenden Tabelle angegeben. Dabei wurde die Temperatur so eingestellt, dass sich vergleichbare Isobutenumsätze ergeben.

In der Reihe zeigt sich deutlich, dass durch die Teilneutralisation des Ionentauschers bei vergleichbaren Umsätzen deutlich verbesserte Selektivitäten zu den C8-Produkten erzielen lassen.

| **Versuch Nr.** | | **2** | **3** | **4** |
|---|---|---|---|---|
| Ionentauscher | | A15 | A15 | A15 |
| Austauschgrad, Metall | | 0% | 10%, Na | 40%, Na |
| Temp. | [°C] | 40.0 | 51.2 | 110.0 |
| Zulauf | [kg/h] | 0.502 | 0.526 | 0.510 |
| | | | | |

| Analyse Zulauf | | | | |
|---|---|---|---|---|
| iso-Butan | [%] | 2.20 | 4.09 | 2.88 |
| n-Butan | [%] | 9.56 | 12.16 | 8.98 |
| trans-Buten | [%] | 9.17 | 8.74 | 8.71 |
| 1-Buten | [%] | 27.71 | 26.56 | 28.33 |
| iso-Buten | [%] | 44.76 | 41.99 | 45.14 |
| cis-Buten | [%] | 6.12 | 5.98 | 5.46 |
| Rest | [%] | 0.49 | 0.46 | 0.49 |
| | | | | |

| Analyse Austrag | | | | |
|---|---|---|---|---|
| iso-Butan | [%] | 2.57 | 4.31 | 3.28 |
| n-Butan | [%] | 10.90 | 13.68 | 10.22 |
| trans-Buten | [%] | 10.43 | 10.14 | 10.43 |
| 1-Buten | [%] | 31.68 | 29.22 | 30.79 |
| iso-Buten | [%] | 37.03 | 35.07 | 37.93 |
| cis-Buten | [%] | 6.88 | 7.06 | 6.87 |
| Rest | [%] | 0.52 | 0.50 | 0.48 |
| | | | | |

| Umsatz iso-Buten | | 27.4% | 25.8% | 26.2% |
|---|---|---|---|---|
| | | | | |
| Selektivitäten | | | | |
| Sel C8 | [%] | 84.9 | 88.68 | 92.66 |
| Sel C12 | [%] | 11.6 | 9.8 | 7.1 |
| Sel C16+ | [%] | 3.5 | 1.31 | 0.07 |
| | [%] | | | |
| 2,4,4-TMP im C8 | | 94.9 | 95.4 | 95.7 |

### Beispiel 5 und 6

In den Versuchen 5 und 6 wurde als Rohstoff jeweils eine Isobuten-haltige Mischung aus C4-Kohlenwasserstoffen eingesetzt. Die Zusammensetzung ist in der nachfolgenden Tabelle widergegeben.

| **Versuch Nr.** | | **5** | **6** |
|---|---|---|---|
| Ionentauscher | | A15 | A15 |
| Austauschgrad, Metall | | 40%, Na | 0%, Na |
| | | | |
| Analyse Zulauf | | | |
| iso-Butan | [%] | 0.01 | 0.03 |
| n-Butan | [%] | 9.25 | 10.82 |
| trans-Buten | [%] | 19.25 | 17.16 |
| 1-Buten | [%] | 11.79 | 14.45 |
| iso-Buten | [%] | 50.72 | 49.55 |
| cis-Buten | [%] | 8.83 | 7.96 |
| Rest | [%] | 0.15 | 0.05 |

Bei Versuch 5 wurden 54 ml eines mit Natriumionen zu 40% teilneutralisierten Ionentauschers des Typs Amberlyst 15 eingesetzt, in Versuch 6 54 ml eines nicht teilneutralisierten Ionentauschers Amberlyst 15 (jeweils getrockneter Ionentauscher). Der Grad des Austausches der Protonen in Versuch 5 wurde durch eine Bestimmung der Kapazität des Ionentauschers überprüft.
Die Versuche wurden analog zu den Beispielen 2 bis 4 durchgeführt.
Bei einer Zulaufmenge von 500 g/h wurde der Umsatz des Isobutens (Umsatz I-Ben) durch Variation der Reaktionstemperatur zwischen 50 und 120°C verändert. Neben dem Umsatz wurden jeweils die Selektivität zu C8-Kohlenwasserstoffen (C8-Sel.) und der Anteil an 2,4,4-Trimethylpentenen (2,4,4-TMP) in der C8-Fraktion gaschromatographisch bestimmt.

| Bilanz-Nr. | Umsatz I-Ben [%] | C8-Sel. [%] | 2,4,4-TMP [%] |
|---|---|---|---|
| **5-a** | **57.69** | **85.33** | **93.60** |
| **5-b** | **63.82** | **81.71** | **93.47** |
| **5-c** | **74.36** | **77.23** | **93.81** |
| | | | |

| Bilanz-Nr. | Umsatz I-Ben 875 | C8-Sel. [%] | 2,4,4-TMP [%] |
|---|---|---|---|
| **6-a** | **57.49** | **78.79** | **94.30** |
| **6-b** | **63.76** | **76.30** | **93.50** |
| **6-c** | **74.66** | **71.61** | **90.43** |

Es zeigt sich, dass bei vergleichbaren Umsätzen an Isobuten mit dem teilneutralisierten Ionentauscher deutlich bessere C8-Selektivitäten erreicht werden können.

## Patentansprüche

1. Verfahren zur Oligomerisierung von Isobuten in Gegenwart von n-Buten an einem festen, sauren Ionenaustauscher,
**dadurch gekennzeichnet**,
dass die Umsetzung an einem Ionenaustauscher durchgeführt wird, dessen acide Protonen teilweise gegen Metallionen ausgetauscht wurden.

2. Verfahren nach Anspruch 1,
**dadurch gekennzeichnet**,
dass 0;1 bis 30 % der aciden Protonen des Ionenaustauschers gegen Metallionen ausgetauscht wurden.

3. Verfahren nach Anspruch 1 oder 2,
**dadurch gekennzeichnet**,
dass ein Gemisch, enthaltend Isobuten, 1-Buten, 2-Buten und Butane zur Oligomerisation eingesetzt wird.

4. Verfahren nach einem der Ansprüche 1 bis 3,
**dadurch gekennzeichnet**,
dass die Metallionen Ionen der Alkalimetalle, Erdalkalimetalle und/oder Seltenen Erden sind.

5. Verfahren nach einem der Ansprüche 1 bis 4,
**dadurch gekennzeichnet**,
dass die Isobuten-haltigen Kohlenwasserstoffmischungen währen der Oligomerisation mindestens teilweise in flüssiger Phase vorliegen.

6. Verfahren nach einem der Ansprüche 1 bis 5,
**dadurch gekennzeichnet**,
dass die Oligomerisation bei 5 bis 160 °C durchgeführt wird.

7. Verfahren nach einem der Ansprüche 1 bis 6,
**dadurch gekennzeichnet**,
dass weniger als 5 Mol-% des bei der Oligomerisation anwesenden 1-Butens zu 2-Buten isomerisiert wird.

8. Verfahren nach einem der Ansprüche 1 bis 7,
**dadurch gekennzeichnet**,
dass der Austrag der Oligomerisierung in C₈-Olefine und C₄-Olefine fraktioniert wird.

9. Verfahren nach Anspruch 8,
**dadurch gekennzeichnet**,
dass das in der C₄-Olefin-haltigen Fraktion enthaltende Isobuten in mindestens einer weiteren Reaktionsstufe mit einem Alkohol verethert wird.

10. Verfahren nach Anspruch 8,
**dadurch gekennzeichnet**,
dass die C₈-Olefin-haltige Fraktion zu den gesättigten Kohlenwasserstoffen hydriert wird.

11. Verfahren zur Herstellung von 1-Buten aus einer C₄-Kohlenwasserstoffmischung, enthaltend Isobuten, 1-Buten, 2-Buten und Butane,
**dadurch gekennzeichnet**,
dass die C₄-Kohlenwasserstoffmischung zur Oligomerisierung von Isobuten in Gegenwart von n-Buten an einem sauren, festen Ionenaustauscher, dessen acide Protone teilweise gegen Metallionen ausgetauscht wurden, umgesetzt, und das 1-Buten aus dem Reaktionsprodukt destillativ abgetrennt wird.

12. Verfahren nach Anspruch 11,
**dadurch gekennzeichnet**,
dass 0,1 bis 30 % der aciden Protonen des Ionenaustauschers gegen Metallionen ausgetauscht wurden.

## Claims

1. Process for oligomerizing isobutene in the presence of n-butene over a solid, acidic ion exchanger,
**characterized in that**
the conversion is carried out over an ion exchanger, some of whose acidic protons have been exchanged for metal ions.

2. Process according to Claim 1,
**characterized in that**
from 0.1 to 30% of the acidic protons of the ion exchanger have been exchanged for metal ions.

3. Process according to Claim 1 or 2,
**characterized in that**
a mixture comprising isobutene, 1-butene, 2-butene and butanes is used for oligomerization.

4. Process according to any of Claims 1 to 3, **characterized in that**
the metal ions are ions of the alkali metals, alkaline earth metals and/or rare earths.

5. Process according to any of Claims 1 to 4, **characterized in that**
the isobutenic hydrocarbon mixtures are at least partially in the liquid phase during the oligomerization.

6. Process according to any of Claims 1 to 5, **characterized in that**
the oligomerization is carried out at from 5 to 160°C.

7. Process according to any of Claims 1 to 6, **characterized in that**
less than 5 mol% of the 1-butene present in the oligomerization is isomerized to 2-butene.

8. Process according to any of Claims 1 to 7, **characterized in that**
the effluent of the oligomerization is fractionated into C₈-olefins and C₄-olefins.

9. Process according to Claim 8,
**characterized in that**
the isobutene present in the C₄-olefinic fraction is etherified with an alcohol in at least one further reaction stage.

10. Process according to Claim 8,
**characterized in that**
the C₈-olefinic fraction is hydrogenated to give the saturated hydrocarbons.

11. Process for preparing 1-butene from a C₄-hydrocarbon mixture comprising isobutene, 1-butene, 2-butene and butanes,
**characterized in that**
the C₄-hydrocarbon mixture for oligomerizing isobutene in the presence of n-butene is converted over an acidic, solid ion exchanger, some of whose acidic protons have been exchanged for metal ions, and the 1-butene is distillatively removed from the reaction product.

12. Process according to Claim 11,
**characterized in that**
from 0.1 to 30% of the acidic protons of the ion exchanger have been exchanged for metal ions.

## Revendications

1. Procédé d'oligomérisation d'isobutène en présence de n-butène sur un échangeur d'ions acide solide, **caractérisé en ce que** la réaction est réalisée sur un échangeur d'ions dont les protons acides ont été partiellement remplacés par des ions métalliques.

2. Procédé selon la revendication 1, **caractérisé en ce que** 0,1 à 30 % des protons acides de l'échangeur d'ions ont été remplacés par des ions métalliques.

3. Procédé selon la revendication 1 ou 2, **caractérisé en ce qu'**un mélange contenant de l'isobutène, du 1-butène, du 2-butène et des butanes est utilisé pour l'oligomérisation.

4. Procédé selon l'une quelconque des revendications 1 à 3, **caractérisé en ce que** les ions métalliques sont des ions de métaux alcalins, de métaux alcalino-terreux et/ou de terres rares.

5. Procédé selon l'une quelconque des revendications 1 à 4, **caractérisé en ce que** les mélanges d'hydrocarbures contenant de l'isobutène se trouvent au moins partiellement en phase liquide pendant l'oligomérisation.

6. Procédé selon l'une quelconque des revendications 1 à 5, **caractérisé en ce que** l'oligomérisation est réalisée à 5 à 160 °C.

7. Procédé selon l'une quelconque des revendications 1 à 6, **caractérisé en ce que** moins de 5 % en moles du 1-butène présent lors de l'oligomérisation est isomérisé en 2-butène.

8. Procédé selon l'une quelconque des revendications 1 à 7, **caractérisé en ce que** la sortie de l'oligomérisation est fractionnée en oléfines en C₈ et oléfines en C₄.

9. Procédé selon la revendication 8, **caractérisé en ce que** l'isobutène contenu dans la fraction contenant les oléfines en C₄ est éthérifié avec un alcool en au moins une étape de réaction supplémentaire.

10. Procédé selon la revendication 8, **caractérisé en ce que** la fraction contenant les oléfines en C₈ est hydrogénée pour former les hydrocarbures saturés.

11. Procédé de fabrication de 1-butène à partir d'un mélange d'hydrocarbures en C₄ contenant de l'isobutène, du 1-butène, du 2-butène et des butanes, **caractérisé en ce que** le mélange d'hydrocarbures en C₄ est mis en réaction pour l'oligomérisation de l'isobutène en présence de n-butène sur un échangeur d'ions acide solide, dont les protons acides ont été partiellement remplacés par des ions métalliques, et le 1-butène est séparé par distillation du produit de réaction.

12. Procédé selon la revendication 11, **caractérisé en ce que** 0,1 à 30 % des protons acides de l'échangeur d'ions ont été remplacés par des ions métalliques.
